(19) Europäisches Patentamt
European Patent Office
Office européen des brevets

(11) **EP 1 967 842 A2**

(12) **EUROPEAN PATENT APPLICATION**

(43) Date of publication:
**10.09.2008 Bulletin 2008/37**

(51) Int Cl.:
*G01N 21/35* (2006.01)  *G01N 21/39* (2006.01)

(21) Application number: **08150520.8**

(22) Date of filing: **22.01.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **23.01.2007 IT BO20070036**

(71) Applicant: **AEA S.r.l.**
**60030 Angeli di Rosora (AN) (IT)**

(72) Inventors:
- **Russo, Adolfo**
  **80063 Piano di Sorrento (IT)**
- **Fioravanti, Matteo**
  **60030 San Marcello (IT)**
- **Romiti, Gino**
  **60024 Filottrano (IT)**

(74) Representative: **Jorio, Paolo et al**
**Studio Torta S.r.l.**
**Via Viotti, 9**
**10121 Torino (IT)**

(54) **Method and device for measuring the concentration of exhaust gases of a boiler**

(57) A device for measuring the concentration (C) of at least one gas species of the exhaust gases of a boiler, this device being equipped with a measuring duct (2) through which the flow (EG) of the exhaust gases leaving the boiler passes; a light source (9) to transmit a light signal (LS) along an optical path (P) extending inside the measuring duct (2) of fixed length (L), this light signal (LS) having a fixed incident intensity (10) at the inlet (7) of the optical path (P); an optical sensor (10) to detect the light signal (LS) at the outlet (8) of the optical path (P); and a signal processing stage (14 and 15) to process, in electrical form, the detected light signal (LS) and provide a processed signal (ES) that represents a measurement of the concentration (C) of the gas species related to the incident intensity value (10) and the length (L) of the optical path (P).

Fig. 1

**Description**

The present invention concerns a method and a corresponding device for measuring the concentration of exhaust gases of a boiler.

In particular, the present invention finds useful, but not exclusive, application in the calibration of the burner of a domestic-type boiler at the end of the manufacturing phase of the boiler, to which the description that follows shall make explicit reference, but without any loss in generality.

Normally, burner calibration of a domestic-type boiler envisages measuring the concentration, or rather the percentage by volume, of at least one gas species of the exhaust gases of the boiler, carbon dioxide ($CO_2$) in particular, and regulating the combustion air to the burner in function of this concentration, and in particular to maximise the concentration of carbon dioxide. A maximum carbon dioxide concentration value essentially corresponds to minimum concentration values for oxygen ($O_2$) and carbon monoxide (CO).

Devices exist on the market for measuring the concentration of exhaust gases of a boiler, these devices comprising a flexible sampling tube, several metres long, which can be connected to an opening made in the flue of the boiler to take a sample of the exhaust gases, an infrared sensor to irradiate the sample with infrared light and detect the light attenuated by the same sample, a treatment unit to treat the sample prior to the sensor taking the reading, and a processing unit to determine the concentration of the gas species of interest, such as carbon dioxide, as a function of the light detected by the sensor.

Treatment of the exhaust gas sample is indispensable for correct operation of the infrared sensor and, in detail, envisages regulating the sample taking flow to sufficiently low levels and cooling the sample to make the water vapour, which is present in the exhaust gases in large amounts, condense and then eliminating it. This treatment involves a considerable slowing down of the entire measuring process: each measurement can in fact require several minutes. These measuring times become extremely onerous in the large-scale production and calibration of standard boilers for domestic use. In addition, the complexity introduced by treatment of the sample reduces the reliability of the measuring device.

The object of the present invention is to provide a method for measuring the concentration of exhaust gases of a boiler and to embody a corresponding measuring device that can be used in the calibration of the burner of said boiler, this method and device being devoid of the above-described drawbacks and, at the same time, of straightforward and economic realization.

In accordance with the present invention, a method and a device are provided for measuring the concentration of at least one gas species of the exhaust gases of a boiler and a method for calibrating a burner of a boiler according to that defined in the enclosed claims.

The present invention shall now be described with reference to the enclosed drawings, which show non-limitative examples of embodiment, where:

- Figure 1 shows a block diagram of the device for measuring the concentration of exhaust gases of a boiler according to the present invention;
- Figure 2 shows, as an axonometric projection, an example of physical embodiment of the measuring device in Figure 1;
- Figure 3 shows a block diagram of a third embodiment of the measuring device of the present invention;
- Figures 4 and 5 show part of the measuring device in Figure 2 according to a fourth embodiment of the present invention;
- Figures 6 and 7 show parts of a block diagram of a fifth embodiment of the measuring device of the present invention; and
- Figure 8 shows, as an axonometric projection, an example of physical embodiment of part of the measuring device in Figure 6.

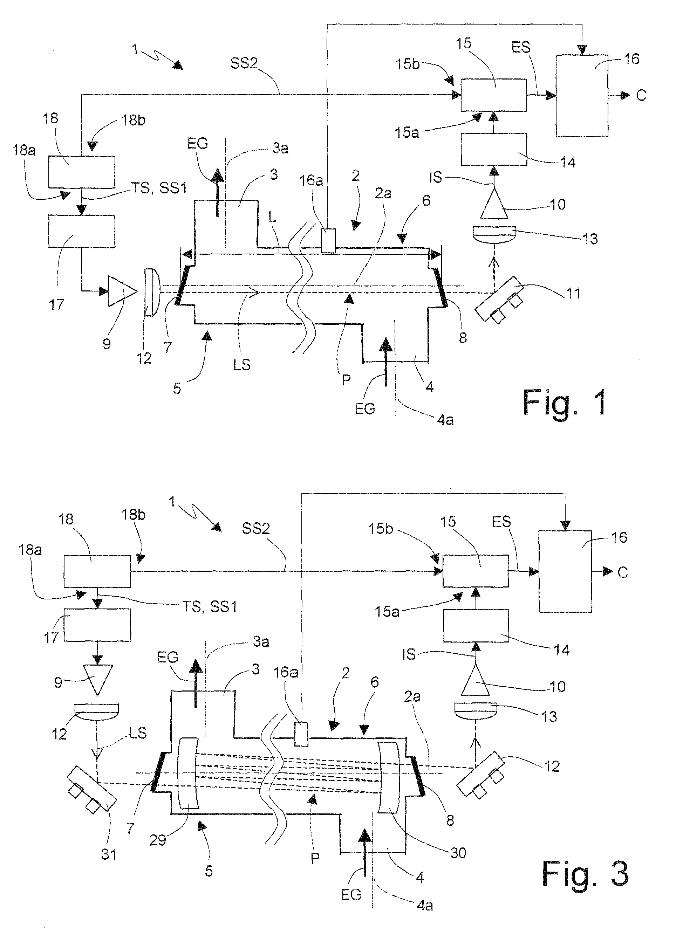

In Figure 1, reference numeral 1 generically indicates, in its entirety, the block diagram of a measuring device for measuring the concentration of at least one gas species, such as carbon dioxide or carbon monoxide, of the exhaust gases of a boiler (not shown) fitted with a flue (not shown) for the discharge of exhaust gases.

The measuring device 1 comprises a measuring duct 2 having a longitudinal axis 2a and two openings 3 and 4 providing lateral access, each of which is made in correspondence to a respective longitudinal end portion 5 and 6 of the measuring duct 2 and having an axis 3a and 4a of symmetry that is transversal, and preferably perpendicular, to axis 2a. One of the openings 3 and 4 is suitable for being fitted directly to the flue to receive a flow EG of exhaust gases from the flue, and the other is suitable for expelling this flow EG after it has traversed the entire measuring duct 2. In correspondence to the end portions 5 and 6, the measuring duct 2 has respective optical windows 7 and 8, which are transversal to axis 2a, and between which a straight optical path P of fixed length L extends inside the measuring duct 2.

The measuring device 1 also comprises a light source consisting of a wavelength-tunable laser diode 9 facing optical window 7 to transmit a light signal along optical path P consisting of a wavelength-modulated laser beam LS having an incident intensity of fixed value IO in correspondence to optical window 7; a detector consisting of a photodiode 10 facing the other optical window 8 to detect the laser beam LS at the end of optical path P and to supply an electrical-type intermediate signal IS; an adjustable mirror 11 to point and centre the received laser beam LS in a precise manner on the photodiode 10 so as to compensate for any mechanical imperfections in construction; an optical collimator 12 inserted between the laser diode 9; and optical window 7 and another optical collimator 13 inserted between the mirror 11 and the photodiode 10. Each optical window 7 and 8 consists of a respective element that is transparent to the wavelengths emitted by the laser diode 9 and has antireflection surfaces for the same wavelengths. In the configuration just described, optical window 7 defines the inlet to the optical path P and optical window 8 defines the outlet of the optical path P, although by construction, the measuring duct 2 could be used in a reversible manner, or rather with optical window 8 as the inlet and optical window 7 as the outlet.

Always with reference to Figure 1, the measuring device 1 comprises a signal amplifier 14 to amplify the intermediate signal IS provided by the photodiode 10; a lock-in amplifier 15 of known type connected to the output of the signal amplifier 14 to receive the amplified intermediate signal IS and provide a processed signal ES obtained by processing the intermediate signal IS in a way to increase the signal-noise ratio related to the light signal LS; and a processing unit 16 connected to the output of the lock-in amplifier 15 to acquire the processed signal ES and configured to determine a value IR for the received intensity of the laser beam LS at the outlet 8 of the optical path P on the basis of the processed signal ES and calculate the concentration C of the gas species as a function of the incident IO and received IR intensities, and length L.

In addition, a thermocouple 16a is mounted on the measuring duct 2 in correspondence to a plane transversal to axis 2a that substantially cuts the optical path P in half and is suitable for measuring the temperature TG of the exhaust gases in the measuring duct 2. The processing unit 16 is configured to calculate the concentration C as a function of the measured temperature TG and a pair of reference parameters, which are a reference concentration Cref of the gas species and a corresponding reference temperature TGref, in order to relate the concentration measurements of the gas species to the reference temperature TGref. This calculation is performed by means of a formula simply inferred from the known ideal gas law. The reference parameters Cref and TGref are determined in a prior calibration phase of the measuring device 1, in which a concentration Cref of the gas species is made to circulate in the measuring duct 2, a measurement of the concentration of the gas species is acquired, the temperature of the gas species is measured, the concentration value Cref is assigned to the acquired measurement of concentration and stored in an internal memory (not shown) of the processing unit 16, and the measured temperature is stored in the internal memory as the reference temperature TGref.

The measuring device 1 also comprises a pilot unit 17 for current piloting the laser diode 9 and a signal generator 18 comprising an output 18a connected to the pilot unit 17 to modulate current to the laser diode 9 for the purpose of modulating the wavelength of the laser beam LS within a band WB of wavelengths comprising a wavelength value WL selected on the basis to the gas species under consideration. For example, in the case where the gas species for which it wished to determine the concentration C is carbon dioxide, the wavelength value WL is 1580 nm. Instead, in the case of carbon monoxide, the wavelength value WL is 2330 nm. The band value WB for the wavelengths is less than or equal to 2 nm.

More in detail, the output 18a of the signal generator 18 is able to provide a modulating signal comprising a sawtooth signal TS to repeat a linear scan of the entire band WB of wavelengths according to a period T of fixed value and, in particular, equal to 10 ms. In consequence, the intermediate signal IS produced by the photodiode 10 represents an optical transmission function of the gas species under consideration within the band WB of wavelengths. This optical transmission function essentially depends on the optical absorption of the gas species, which follows the Lambert-Beer Law and in the case under consideration can be described by the following formula:

$$I(\nu) = I0 \cdot \exp\{-\alpha(\nu) \cdot C \cdot L\}, \qquad (1)$$

where $\alpha(\nu)$ is a function that defines the optical absorbance of the gas species as the wavelength $\nu$ of the incident light signal LS varies and $I(\nu)$ is the intensity of the light signal LS at outlet 8 of the optical path P at wavelength $\nu$. The values of the function $\alpha(\nu)$ as the wavelength $\nu$ varies can be obtained from known tables and databases.

The signal generator 18 produce a further wavelength modulation of the laser beam LS, and in particular a sinusoidal modulation, that in combination with a corresponding demodulation operated by the lock-in amplifier 15, allows the above-mentioned signal-noise ratio to be increased on the signal represented by the transmitted laser beam LS.

In particular, the modulating signal comprises a first sinusoidal signal SS1 oscillating at a frequency F1 of fixed value, and in particular equal to 100 kHz, superimposed on the sawtooth signal TS. The signal generator 18 comprises an additional output 18b able to provide a second sinusoidal signal SS2 in phase with the first sinusoidal signal SS1 and oscillating at a frequency F2 equal to twice frequency F1. The lock-in amplifier 15 comprises an signal input 15a to receive the amplified intermediate signal IS and a reference input 15b to receive sinusoidal signal SS2 so that the lock-in amplifier 15 can synchronize itself with sinusoidal signal SS1 to carry out a second derivate discrimination according to a technique known as "Phase Sensitive Detection". The result is that the processed signal ES corresponds to the second derivate of the envelope of the intermediate signal IS and thus represents a measurement of the concentration C related to the specific incident intensity value 10 and the specific length value L of the optical path P.

The processing unit 16 is configured to determine the received intensity value IR on the basis of the processed signal ES, and in particular to determine the value IR as the peak value of the processed signal ES. Lastly, the processor 16 calculates the concentration C via relation (1), setting I(v) equal to the received intensity value IR.

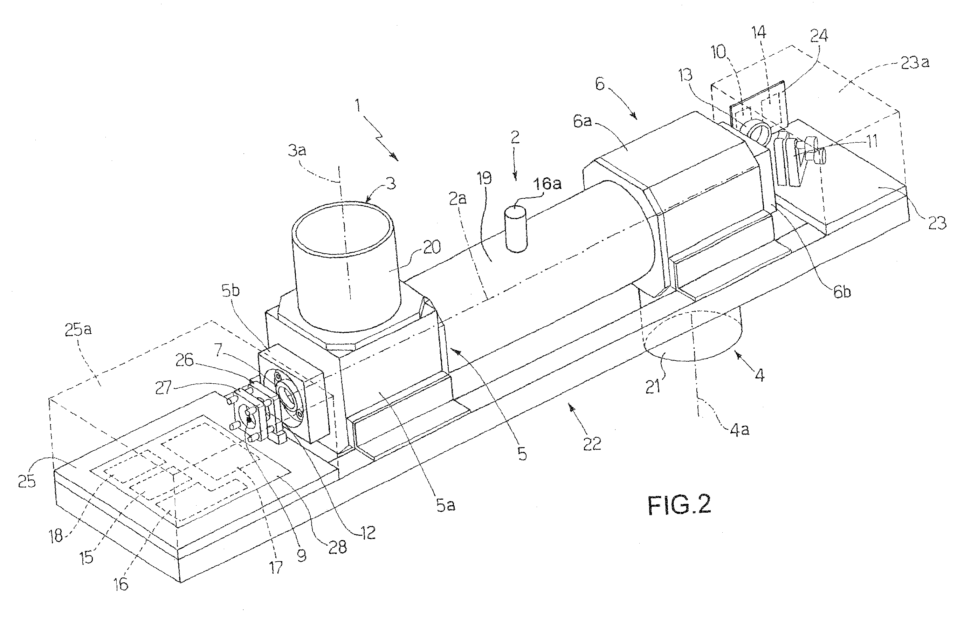

Figure 2 shows an example of embodiment of the measuring duct 2 and the location of the various components of the measuring device 1 described above with respect to the measuring duct 2.

The measuring duct 2 comprises a central pipe 19 of fixed diameter, for example 10 cm, and two end pipes 20 and 21, which are of the same type as the central pipe 19 and define the openings 3 and 4. The longitudinal end portions 5 and 6 of the measuring duct 2 are formed by respective hollow connecting units 5a and 6a suitable for connecting the end pipes 20 and 21 with the central pipe 19. Each connecting unit 5a and 6a has a through hole (not shown) coaxial with axis 2, this through hole communicating at one end with the central pipe 19 and closed at the opposite end by a respective optical window 7 and 8. In particular, each connecting unit 5a and 6a is provided with a respective support 5b and 6b positioned in correspondence to the through hole to support the associated optical window 7 and 8 so that the latter closes the hole. Only optical window 7 is visible in Figure 2. The measuring duct 2 is mounted on a frame 22 comprising a first platform 23, on which the mirror 11, the collimator 13 and an printed circuit board 24 including the photodiode 10 and the signal amplifier 14 are mounted, and a second platform 25, on which a support 26 for the collimator 12 is mounted, together with a support 27 for the laser diode 9 mounted at an adjustable distance from support 26 in order to focus the laser beam LS on the mirror 11, and a printed circuit board 28 including the pilot unit 17, signal generator 18, lock-in amplifier 15, and processing unit 16. The platforms 23 and 25 have respective covers 23a and 25a fitted on them, indicated by dashed lines in Figure 2, to protect the components mounted on the platforms 23 and 25.

In a second embodiment, not shown, of the measuring device 1 according to the present invention, the processing unit 16 is not integrated in either of the two printed circuit boards 24 and 28, but is constituted by an external processor, such as an opportunely programmed personal computer equipped with a special acquisition unit connected to the output of the lock-in amplifier 15.

The length of the measuring duct 2 measured along axis 2a is determined by a compromise between the maximum desired bulk of the measuring device 1 and the length L of the optical path P required to guarantee sufficient sensitivity of the measuring device 1 itself. For a greater length L, there is correspondingly greater optical absorption, and therefore greater sensitivity for the measuring device 1. Furthermore, the relation (1) establishes that for the same sensitivity and concentration C, an optical path P of longer length L is required for low absorbance values $\alpha(v)$.

Figure 3 shows the block diagram of a third embodiment of the measuring device 1 according to the present invention, this measuring device 1 differing from that shown in Figure 2 in that it includes two reflecting units 29 and 30, each housed in a respective of the connecting unit 5a and 6a, and an adjustable mirror 31 to point the laser beam LS through the optical window 7 so as to intercept a first reflecting unit 29 at an angle on incidence such that multiple reflections are generate between the reflecting units 29 and 30 that multiply the length L of the optical path P, which thus becomes composed of a series of rectilinear segments. In this way, it is possible to contain the length of the measuring duct 2, for example to a value between 30 and 50 cm, whilst still maintaining sufficient sensitivity even for gas species with low absorbances $\alpha(v)$.

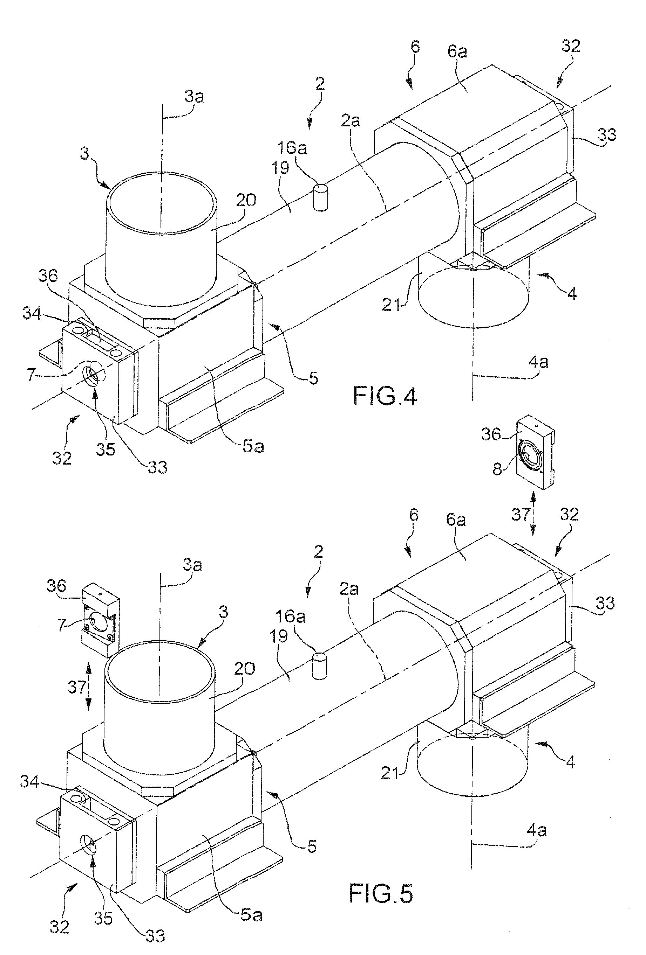

According to a fourth embodiment of the present invention shown in Figures 4 and 5, the optical windows 7 and 8 can be removed to allow cleaning. In fact, after a few weeks of continuous work, a thin film of fine particles deposits on the optical windows 7 and 8, limiting the passage of the laser beam LS.

With special reference to Figures 4 and 5, the measuring duct 2 comprises two identical closure devices 32, each of which is mounted on a respective connecting unit 5a and 6a to close the associated through hole in substitution of the associated support 5b and 6b in Figure 2 and is able to house a respective optical window 7 and 8 in an extractable manner. In Figures 4 and 5, only closure device 32 housing optical window 7 and mounted on connecting unit 5a is completely visible.

In detail, each closure device 32 comprises a plate 33, which is mounted on the connecting unit 5a and 6a transversally to axis 2a, has a cavity 34 open to the outside, and a hole 35 substantially coaxial with axis 2a to allow passage of the laser beam. Each closure device 32 also comprises a support element 36 able to support the associated optical window 7 and 8 and couples with the cavity 34 in an extractable manner. More precisely, support 36 is mobile with respect to the plate 33 along a direction 37 (Figure 5) perpendicular to axis 2a, to and from an operative coupling position of the same support 36 with the cavity 34.

Figure 4 shows the closure devices 32 with the respective supports 36 in the operative position, in which the associated optical windows 7 and 8 each remain completely inside the respective cavity 34, in a position substantially coaxial with axis 2a and the associated hole 35, to allow normal operation of the measuring device 1. Instead, Figure 5 shows the closure devices 32 with the respective supports 36 moved away from the operative position so as to allow easy inspection and cleaning, or rapid substitution of the relevant optical windows 7 and 8.

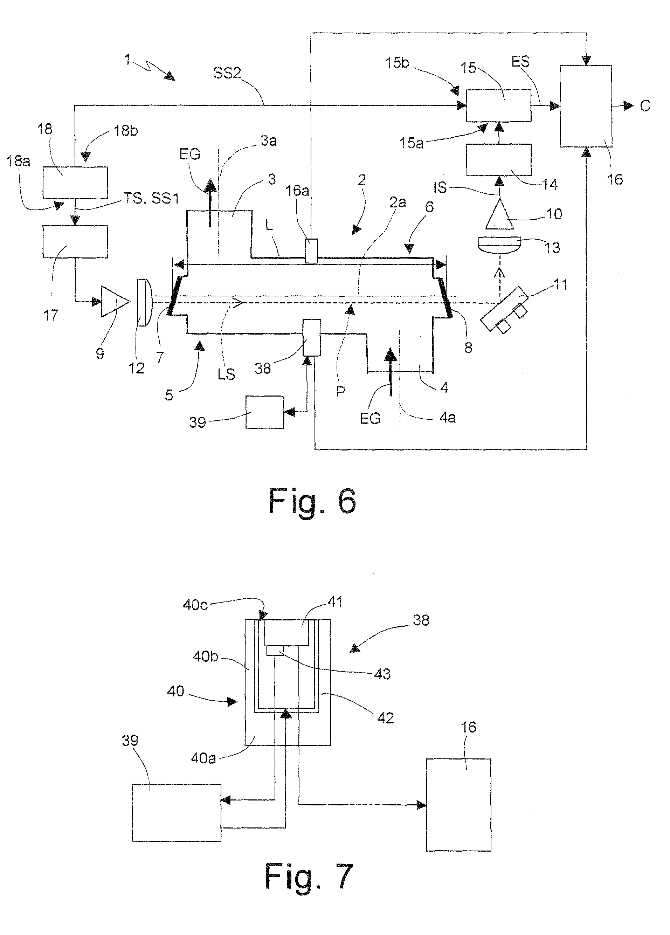

Figures 6, 7 and 8 show a fifth embodiment of the present invention that is particularly advantageous for condensation boilers, the exhaust gases of which have temperatures that vary considerably along the flue, due to the substantial change in the percentage of water in gaseous form.

With reference to Figure 6, in which corresponding elements are indicated with the same reference numerals of Figure 1, the measuring device 1 comprises a water sensor 38, which is located on the measuring duct 2 in correspondence to a plane transversal to axis 2a that substantially cuts the optical path P in half and is connected to the processing unit 16 to provide it with measurements of the concentration of water in gaseous form halfway along the optical path P, and a thermoregulator 39 to keep the water sensor 38 at a constant temperature.

Figure 7 shows the water sensor 38 in greater detail and its connection to the thermoregulator 39. The water sensor 38 comprises a cup body 40, which has an insulated bottom wall 40a, an insulated side wall 40b and an opening 40c opposite to the bottom wall 40a. The water sensor 38 also comprises a plurality of components housed in the cup body 40, such as a commercial-type relative humidity sensor 41 in correspondence to the opening 40c, a resistor 42 to heat the humidity sensor 41 and a temperature sensor 43 to provide measurements of the temperature of the humidity sensor 41.

The thermoregulator 39 is connected to the resistor 42 to electrically power it and to the temperature sensor 43 to receive temperature measurements from it and is able to adjust the electrical power supply to the resistor 42 based on these temperature measurements to keep the humidity sensor 41, and therefore the water sensor 38, at the dew point temperature corresponding to a preset concentration of water in gaseous form that is determined on the basis of a desired concentration for other gas species, such as carbon dioxide, produced by the combustion reaction in a stoichiometric regime. This temperature is henceforth indicated as Tden.

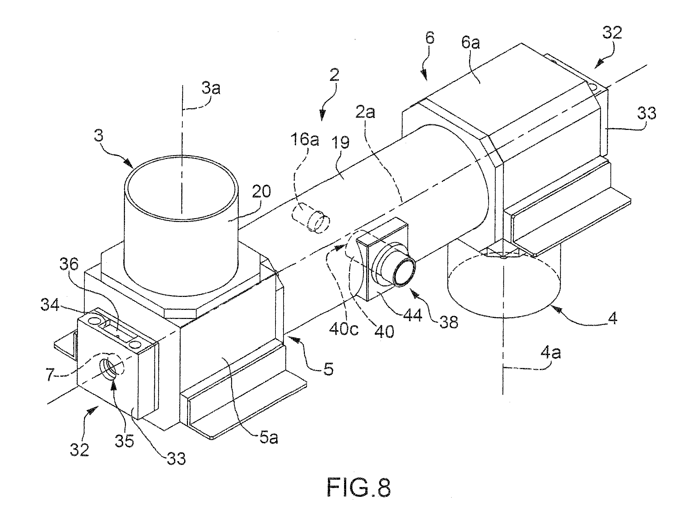

With special reference to Figure 8, in which corresponding elements are indicated with the same reference numerals of Figure 4, the water sensor 38 is mounted on the measuring duct 2 with the cup body 40 inserted in a hole (not shown) made in the central pipe 19, substantially halfway along its length and in a way that a portion of the cup body 40 comprising the opening 40c projects inside the same central pipe 19. The cup body 40 is fixed to the central pipe 19 by a specially provided support 44 having a surface shaped to match the curvature of the central pipe 19.

Considering the specific case of a natural gas powered boiler, the combustion reaction theoretically produces water and carbon dioxide in gaseous form. In this case, temperature Tden is chosen from a range delimited by a minimum temperature Tmin of 58 °C, which coincides with the minimum dew point temperature for having a target carbon dioxide concentration of 9%, and a maximum temperature Tmax of 85 °C, which coincides with the maximum temperature at which the chosen humidity sensor 41 can work. Preferably, temperature Tden is equal to 65 °C, which is the best compromise between the two requirements mentioned above.

The water sensor 38 is pre-calibrated to provide absolute concentration measurements of water in gaseous form. Calibration of the water sensor 38 is carried out before mounting it on the measuring duct 2 and consists in maintaining the humidity sensor 41 at a constant temperature inside a climatic chamber, generating a series of known concentrations of water in gaseous form inside the climatic chamber, measuring, for each generated water concentration, a corresponding electrical voltage provided by the humidity sensor 41, and storing the correspondence between the generated water concentrations and measured output voltages, in the form of a look-up table, in an internal memory (not shown) of the processing unit 16.

In accordance with said fifth embodiment of the present invention, the processing unit 16 uses the measurements of absolute water concentration in the measuring duct 2 provided by the water sensor 38 to determine a so-called "dry" measurement of the concentration of the gas species under consideration. In fact, the concentration measurement C calculated with formula (1) is affected by the water concentration in the measuring duct 2, and so should be considered as a so-called "wet" measurement. The processing unit 16 is therefore configured to detect the electrical voltage provided by the water sensor 38 and convert it into an absolute water concentration measurement via the look-up table, created as previously described, and to calculate, by means of a formula that depends on the combustion reaction involved, a concentration measurement C of the gas species based on the wet concentration calculated with formula (1) and the absolute water concentration. In the case of natural gas combustion, the dry concentration of carbon dioxide, henceforth indicated as C02dry(%), is calculated as a function of the wet concentration of carbon dioxide, henceforth indicated as CO2wet (%), and the water concentration H2O (%) by means of the formula:

```
CO2dry(%) = CO2wet(%) / (1- H2O(%)/100).                    (2)
```

According to a further embodiment of the present invention, the measuring device 1 comprises the measuring duct 2 shown in Figure 2, or rather the measuring duct 2 devoid of the closure devices 32, and the water sensor 38 described above and shown in Figures 6 to 8 mounted on the central pipe 19.

The main advantage of the above-described measuring device 1 is to allow extremely rapid measurements of the concentration C of a gas species of the exhaust gases of a boiler. In particular, it is possible to perform up to 100 measurements per second. With respect to the instruments known of up until now for the calibration of domestic-type boilers, the substantial increase in measuring speed allows measurement samples to be collected in less time and to then extract more accurate and stable average values. In addition, the marked constructional simplicity guarantees considerable sturdiness and reliability. Finally, the variant comprising the water sensor 38 is particularly advantageous for a condensation boiler, as it allows precise measurements to be obtained of the concentration C of a gas species of the exhaust gases independently of the water concentration in the measuring duct 2.

**Claims**

1. Method for measuring the concentration of at least one gas species of the exhaust gases of a boiler, the method being **characterized in that** it comprises the phases of:

   - transmitting a monochromatic light signal (LS) along an optical path (P) extending inside at least one segment of a flow (EG) of the exhaust gases leaving the boiler and having an inlet (7), an outlet (8) and a length, measured between said inlet (7) and outlet (8), of fixed value (L), this light signal (LS) presenting an incident intensity of fixed value (10) at the inlet (7) of the optical path (P),
   - detecting the light signal (LS) at the outlet of the optical path (P),
   - determining a received intensity value (IR) of the light signal (LS) detected at the outlet (8) of the optical path (P), and
   - calculating the concentration (C) of the gas species as a function of the values of incident (IO) and received (IR) intensity of the light signal (LS) and the length (L) of the optical path (P).

2. Method according to claim 1, in which said transmission of the light signal (LS) provides for modulating the wavelength of this light signal (LS) within a band of wavelengths (WB) including a wavelength value (WL) selected according to said gas species, said discrimination of the light signal (LS) producing an intermediate signal (IS) representing an optical transmission function of said gas species in this band of wavelengths (WB).

3. Method according to claim 2, in which said wavelength is modulated with a sawtooth signal (TS).

4. Method according to claim 3, in which said wavelength is modulated with a first sinusoidal signal (SS1) superimposed on said sawtooth signal (TS), said determination of the received intensity value (IR) of the light signal (LS) comprising the phases of:

   - supplying a processed signal (ES) by effecting on said intermediate signal (IS) a synchronized lock-in amplification with the first sinusoidal signal (SS1), and
   - determining the received intensity value (IR) as a function of the processed signal (ES).

5. Method according to claim 4, in which said first sinusoidal signal (SS1) oscillates at a first frequency (F1), said lock-in amplification being synchronized on a second sinusoidal signal (SS2) in phase with the first sinusoidal signal (SS1) and oscillating at a second frequency (F2) equal to twice that of the first frequency (F1) to carry out a second derivate discrimination of the light signal (LS).

6. Method according to any of claims 1 to 5, in which said light signal consists of a laser beam (LS).

7. Method according to any of claims 2 to 6, in which said at least one gas species is carbon dioxide; said wavelength value (WL) is equal to 1580 nm.

8. Method according to any of claims 2 to 6, in which said at least one gas species is carbon monoxide; said wavelength value (WL) is equal to 2330 nm.

9. Method according to any of claims 1 to 8, in which said gas species has an optical absorbance that is variable in function of said wavelength of the light signal (LS), said length value (L) of said optical path (P) being determined on the basis of the optical absorbance.

10. Method according to any of claims 1 to 9, comprising:

- determining an absolute water concentration measurement at a point of said optical path (P) by means of a humidity sensor (41),

said concentration (C) of the gas species being calculated as a function of the measured absolute water concentration.

11. Method according to claim 10, in which determining absolute water concentration measurements at a point of said optical path (P) by means of the humidity sensor (41) comprises:

- calibrating the humidity sensor (41) by generating a series of known water concentrations at a constant temperature, measuring the corresponding electrical voltages provided by the humidity sensor (41) and storing the correspondence between the known generated water concentrations and the measured electrical voltages,
- positioning the humidity sensor (41) at said point on the optical path (P),
- maintaining the humidity sensor (41) at a constant temperature with a fixed value (Tden), and
- determining the absolute water concentration measurement by processing the electrical voltage provided by the humidity sensor (41) via said correspondence produced during calibration.

12. Method according to claim 11, in which the value (Tden) of said constant temperature is between a first temperature (Tmin) and a second temperature (Tmax) greater than the first temperature (Tmin), the first temperature (Tmin) being equal to a minimum dew point temperature to have a target concentration for said gas species and the second temperature (Tmax) being equal to the maximum temperature at which said humidity sensor (41) can work.

13. Method according to any of claims 1 to 12, comprising:

- measuring the temperature (TG) of the gas species at a point on said optical path (P), and
- calculating said concentration (C) of the gas species as a function of the temperature of the gas species (TG), a reference concentration (Cref) of the gas species and a reference temperature (TGref) of the gas species.

14. Method for calibrating a burner of a boiler, this method comprising the phases of:

- measuring the concentration (C) of at least one gas species of the exhaust gases of the boiler, and
- regulating the combustion air for the burner according to this concentration (C),

the method being **characterized in that** the phase of measuring the concentration (C) of at least one gas species is defined by any of claims 1 to 13.

15. Device for measuring the concentration of at least one gas species of the exhaust gases of a boiler comprising a flue for the discharge of the exhaust gases, the device (1) being **characterized in that** it comprises at least one measuring duct (2) suitable for being traversed by a flow (EG) of exhaust gases arriving from the flue, a light source (9) to transmit a light signal (LS) along an optical path (P) extending inside the same measuring duct (2), which has an inlet (7), an outlet (8) and a length, measured between said inlet (7) and outlet (8), of fixed value (L), this light signal presenting an incident intensity of fixed value (IO) at the inlet (7) of the optical path, optical detection means (10) for detecting the light signal (LS) at the outlet (8) of the optical path (P), and signal processing means (14 and 15) for processing, in electrical form, the detected light signal (LS) and providing a processed signal (ES) that represents a measurement of the concentration (C) of the gas species associated with the incident intensity value (IO) and the length value (L) of the optical path (P).

16. Device according to claim 15, comprising acquisition and processing means (16) connected to the output of said signal processing means (14 and 15) and configured to acquire said processed signal (ES), determine a received intensity value (IR) for said light signal (LS) at the outlet (8) of said optical path (P) as a function of the processed signal (ES) and determine said concentration measurement (C) of the gas species as a function of incident (IO) and received (IR) intensity values of the light signal (LS) and the length (L) of the optical path (P).

17. Device according to claim 15 or 16, in which said light source (9) can be tuned in wavelength, the device (1) comprising a signal generator (18) able to provide a modulating signal to modulate the wavelength of the light signal (LS) within a band of wavelengths (WB) including a wavelength value (WL) selected according to said gas species.

18. Device according to claim 17, in which said optical detection means (10) produce an intermediate signal (IS) representing an optical transmission function of said gas species in said band of wavelengths (WB).

19. Device according to claim 17 or 18, in which said modulating signal comprises a sawtooth signal (TS).

20. Device according to claim 19, in which said modulating signal comprises a first sinusoidal signal (SS1) superimposed on said sawtooth signal (TS), said signal processing means (14 and 15) comprising a lock-in amplifier (15), which is synchronized with the first sinusoidal signal (SS1), receives the intermediate signal (IS) and processes it to provide said processed signal (ES).

21. Device according to claim 20, in which said first sinusoidal signal (SS1) oscillates at a first frequency (F1), said signal generator (18) providing a second sinusoidal signal (SS2) to said lock-in amplifier (15) in phase with the first sinusoidal signal (SS1) and oscillating at a second frequency (F2) equal to twice that of the first frequency (F1).

22. Device according to claim 20 or 21, in which said signal processing means (14 and 15) comprise a signal amplifier (14) connected between said optical detection means (10) and said lock-in amplifier (15).

23. Device according to any of claims 15 to 22, in which said light source comprises a laser diode (9) and said optical detection means comprise a photodiode (10).

24. Device according to any of claims 17 to 22, in which said light source comprises a laser diode (9) and said optical detection means comprise a photodiode (10), said signal generator (18) being able to provide a modulating signal to modulate the current to the laser diode (9) so as to modulate said wavelength of the light signal (LS) within said band of wavelengths (WB).

25. Device according to any of claims 15 to 24, in which said gas species has a variable optical absorbance depending on said wavelength of the light signal (LS), said length value (L) of the optical path (P) being determined on the basis of the optical absorbance.

26. Device according to any of claims 15 to 25, in which said measuring duct (2) comprises at least one first opening (3) able to be fitted directly to said flue to receive said flow (EG) of exhaust gases, and at least a second opening (4) for expelling this flow (EG) after the same flow (EG) has traversed the measuring duct (2).

27. Device according to any of claims 15 to 26, in which said measuring duct (2) has a longitudinal axis (2a) and said openings (3 and 4) have respective axes (3a and 4a) transversal to the longitudinal axis (2a).

28. Device according to claim 26 or 27, in which said measuring duct (2) has two longitudinal end portions (5 and 6), each of said openings (3 and 4) being made laterally in the measuring duct (2) in correspondence to a respective longitudinal end portion (5 and 6).

29. Device according to claim 28, in which said measuring duct (2) has a longitudinal axis (2a) and comprises two optical windows (7 and 8), each placed in respective correspondence to said longitudinal end portions (5 and 6) so as to be transversal to the longitudinal axis (2a), with one (7) of the optical windows (7 and 8) defining said inlet of said optical path (P) and the other (8) defining said outlet of the optical path (P).

30. Device according to claim 29, in which each of said longitudinal end portions (5 and 6) has a respective through hole coaxial with said longitudinal axis (2a), said measuring duct (2) comprising means of closure (32) mounted on the longitudinal end portions (5 and 6) to close the through holes and suitable for housing the optical windows (7 and 8) in an extractable manner.

31. Device according to any of claims 15 to 30, comprising first reflecting means (11) able to point the received light signal (LS) to centre it on the optical detection means (10) in order to compensate for any mechanical imperfections in construction of the device (1) itself.

32. Device according to any of claims 15 to 31, comprising a first optical collimator (12) placed at the outlet of said light source (9) and a second optical collimator (13) placed at the inlet of said optical detection means (10).

**33.** Device according to any of claims 15 to 32, in which said optical path (P) comprises at least one rectilinear segment.

**34.** Device according to any of claims 15 to 33, and comprising second reflecting means (29, 30 and 31) to generate multiple reflections inside said measuring duct (2) in order to define an optical path comprising a series of rectilinear segments.

**35.** Device according to claim 34, in which said measuring duct (2) has two longitudinal end portions (5, 6), said second reflecting means (29, 30 and 31) comprising two reflecting units (29 and 30), each housed in a respective longitudinal end portion (5 and 6), and third reflecting means (31) for pointing said light signal (LS) to the inlet (7) of said optical path (P) so that the same light signal (LS) intercepts the first (29) of the reflecting units at an angle of incidence that generates said multiple reflections.

**36.** Device according to any of claims 16 to 35, comprising water sensor means (38) placed in said measuring duct (2) and connected to said acquisition and processing means (16) to provide them with an absolute water concentration measurement at a point on said optical path (P), and thermoregulation means (39) connected to the water sensor means (38) to maintain them at a constant temperature, the acquisition and processing means (16) being configured to determine said concentration measurement (C) of the gas species as a function of the absolute water concentration measurement.

**37.** Device according to claim 36, in which said water sensor means (38) comprise humidity sensor means (41), heater means (42) for heating the humidity sensor means (41) and temperature measurement means (43) to provide measurements of the temperature of the humidity sensor means (41), said thermoregulation means (39) being able to control the heater means (42) on the basis of the measurements provided by the temperature measurement means (43) to keep the humidity sensor means (41) at a constant temperature.

**38.** Device according to any of claims 16 to 37, comprising temperature measurement means (16a) placed in said measuring duct (2) and connected to said acquisition and processing means (16) to provide them with a measurement of the temperature (TG) of said gas species at a point on said optical path (P), the acquisition and processing means (16) being configured to determine said concentration measurement (C) of the gas species as a function of the temperature measurement (TG) of the gas species.

Fig. 1

Fig. 3

FIG.2

FIG.4

FIG.5

Fig. 6

Fig. 7

FIG.8